# EUROPEAN PATENT SPECIFICATION

(11) **EP 0 487 768 B1**
(45) Date of publication and mention of the grant of the patent: **25.01.1995**
(21) Application number: 90122693.6
(22) Date of filing: 28.11.1990
(51) Int. Cl.: A61K 9/14, A61K 9/16, A61K 9/18, A61K 9/20, A61K 31/50

(54) **Pharmaceutical composition with improved dissolution property**
Arzneimittel mit verbesserter Auflösungsgeschwindigkeit
Composition pharmaceutique à vitesse de dissolution améliorée

(43) Date of publication of application: 03.06.1992
(73) Proprietor: YOSHITOMI PHARMACEUTICAL INDUSTRIES, LTD., Osaka-shi Osaka 541 (JP)
(72) Inventor: Ogawa, Kenji, Chikujo-gun, Fukuoka 871-09 (JP); Shibata, Toshiyuki, Nakatsu-shi, Oita 871 (JP); Samemoto, Hirofumi, Nakatsu-shi, Oita 871 (JP)
(74) Representative: von Kreisler, Alek, Dipl.-Chem.

(56) References cited:
- EP-A- 0 281 045
- CHEMICAL PHARMACEUTICAL BULLETIN, vol. 36, no. 7, 1988, pages 2562-2569,Pharmaceutical Society of Japan, Tokyo, JP; T. ISHIZAKI et al.: "Complexationof aspirin with potato starch and improvement of dissolution rate by drymixing"

## Description

### BACKGROUND OF THE INVENTION

The present invention relates to pharmaceutical compositions with improved dissolution property which contain benzothiepino[5,4-c]pyridazine compounds represented by the formula 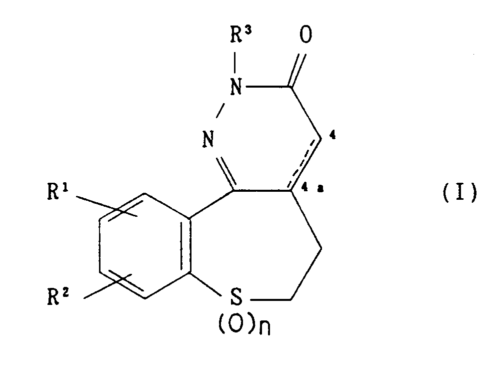
wherein each symbol is as defined below, which are useful as a pharmaceutical agent such as an antianxiety drug, or the like.

As shown in EP-A-281045, the benzothiepino[5,4-c]pyridazine compound of the formula (I) is a selective antianxiety drug with less side effects such as lowering of attention, concentration, reflex movement, etc., sleepiness, dizziness, or the like which benzodiazepine compounds represented by diazepam accompany.

The specification of said EP-A-281045 discloses formulation examples of 10 mg tablets (total amount 120.0 mg) of Compound (I) [a compound of the formula (I)] (10.0 mg), lactose (58.5 mg), corn starch (25.0 mg), crystalline cellulose (20.0 mg), polyvinylpyrrolidone K-30 (2.0 mg), talc (4.0 mg) and magnesium stearate (0.5 mg).

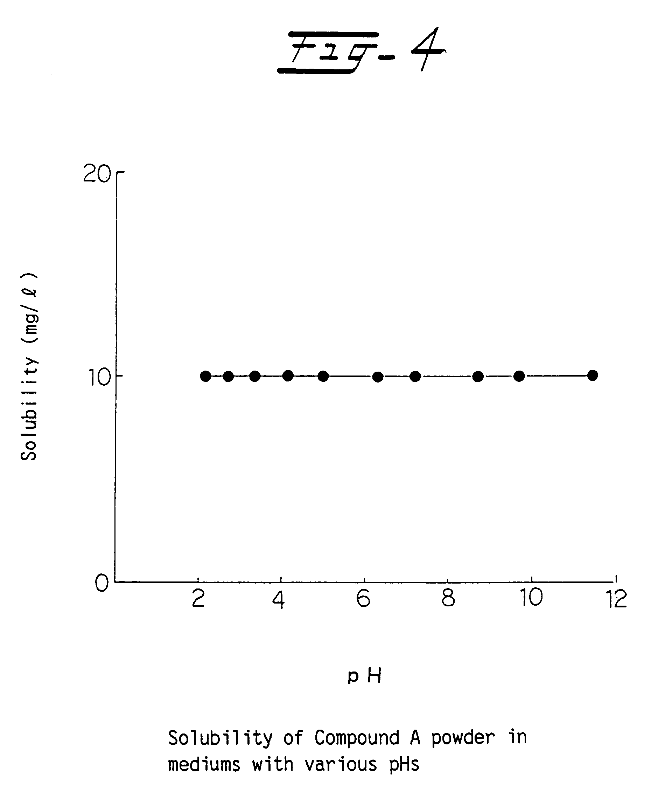

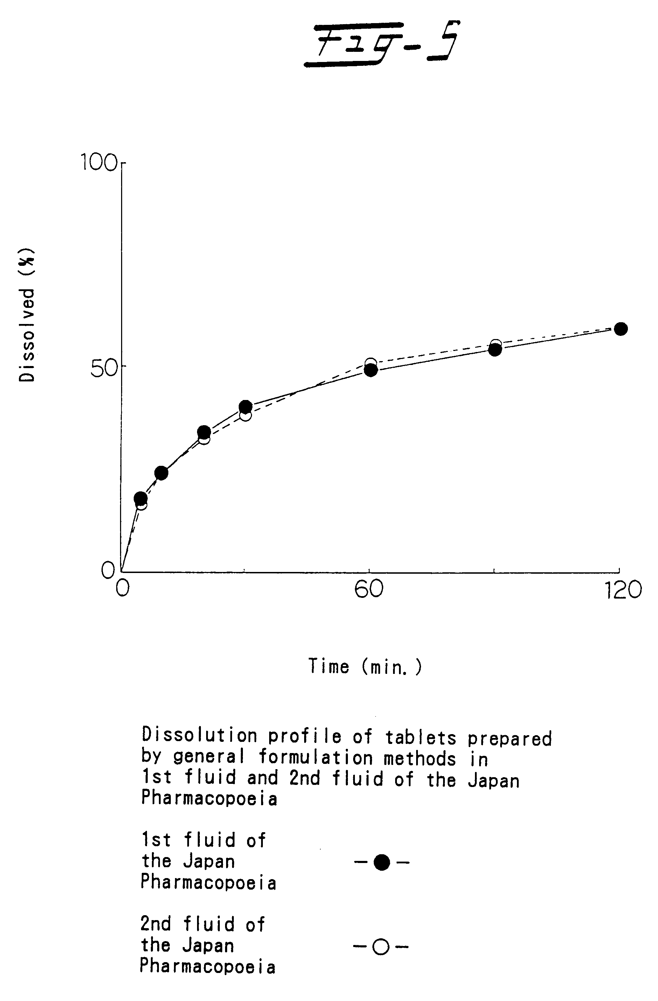

Examination of the solubility of 2-(4-chlorophenyl)-5,6-dihydro-(1)benzothiepino[5,4-c]pyridazine-3(2H)-one 7-oxide (hereinafter referred to as Compound A), one of Compounds (I), shows that the compound is slightly soluble in mediums with various pHs including water, which is shown in Figure 4. Thus, as shown in Fig. 5 in the results of the dissolution test of the tablets prepared by a conventional means employed for formulation of preparations, such as mixing Compound A with lactose, corn starch, etc. for granulation, adding talc, magnesium stearate, etc. and compressing same, the dissolution property is not entirely satisfactory, reflecting the properties of the compound per se.

The tablets with such dissolution property have, when administered to humans, possibilities of exerting a bad influence on absorption ratio or absorption rate via digestive tracts. In order to secure uniform, quick pharmaceutical effects on target patients, a pharmaceutical composition with improved dissolution property should be developed. That is, it is desirable to prepare a pharmaceutical composition with the improved dissolution property of the Compound by adding additives in combination as a carrier, which do not exhibit any particular pharmacological actions by themselves and are harmless, followed by formulation processes.

As the methods for improving dissolution of slightly-soluble pharmaceuticals, there are known (1) a method of finely-powdering, (2) a method of forming a molecular compound (3) a method of forming a solid dispersion and (4) a method of converting into a soluble derivative, and the like. However, there has not been found sufficient dissolution-improving effects by a known method belonging to (1). When tablets, etc. are prepared according to the methods belonging to the above methods (2) and (3), effects cannot be expected unless the additives are added in a not less than several times larger amount relative to the amount of the bulk of the Compound. Thus, they pose problems when put to practical use in view of the large size of the dosage form of the Compound. Furthermore, the above method (4) cannot be adopted in case where it is required to use the Compound per se.

Further, it has been disclosed that the complex powder prepared by mixing aspirin and potato starch by a centrifugal rotating mixer (aspirin content 5.6%) gives high dissolution rate as compared with a physical mixture [Chem. Pharm. Bull., *36* , 2562-2569 (1988)]. However, said aspirin-containing complex powder merely improves dissolution rate of aspirin and does not improve solubility of aspirin per se. In addition, the complex powder contains a large amount of starch, giving rise to problems in actual application thereof to pharmaceutical preparations in view of their bulky form.

### BRIEF DESCRIPTION OF THE DRAWINGS

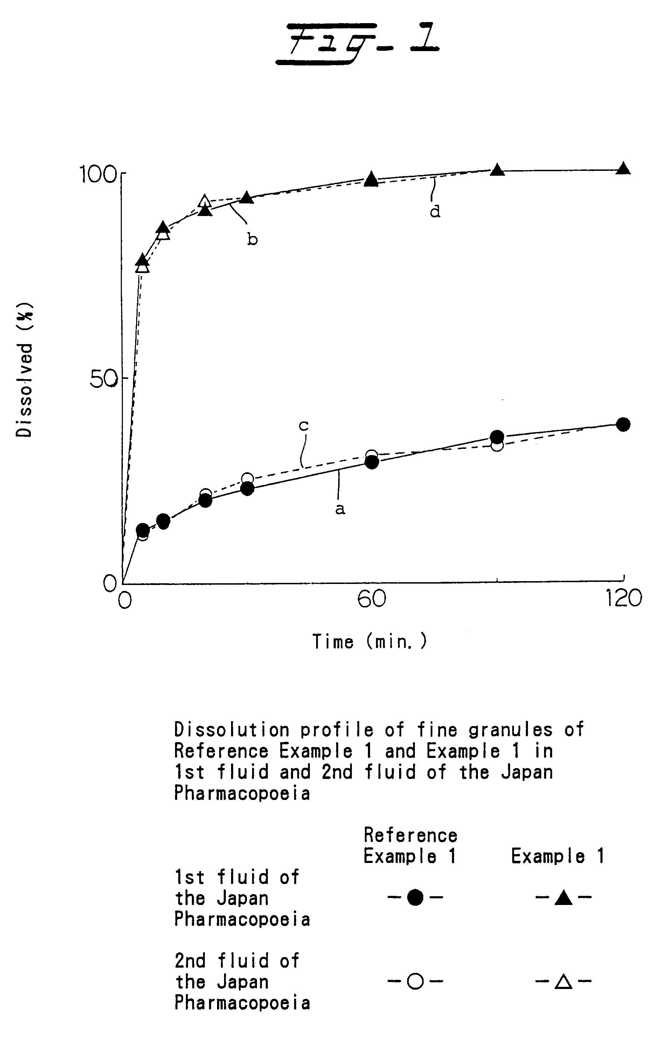

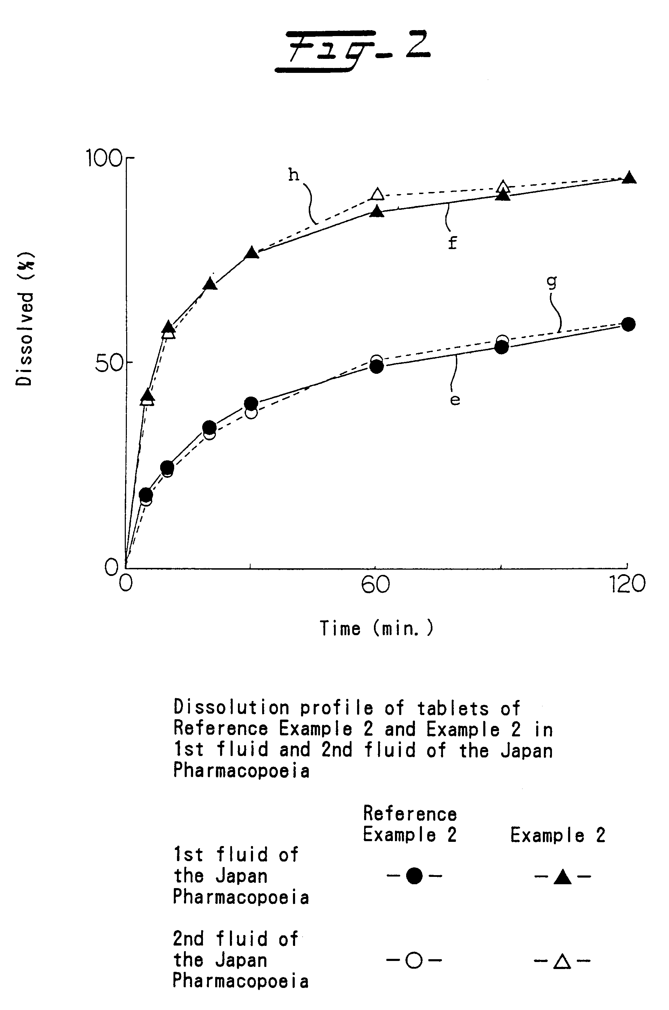

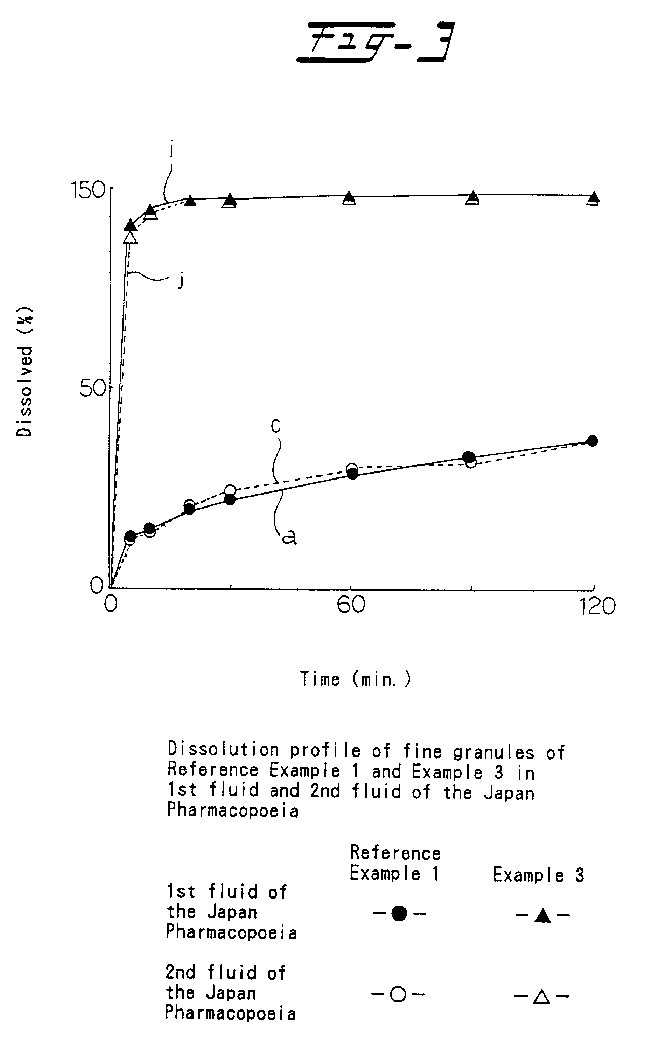

Fig. 1 is a graph showing the results of the dissolution test of the fine granules of Reference Example 1 and Example 1 in the 1st fluid and the 2nd fluid, Fig. 2 is a graph showing the results of the dissolution test of the tablets of Reference Example 2 and Example 2 in the 1st fluid and the 2nd fluid, Fig. 3 is a graph showing the results of the dissolution test of the fine granules of Reference Example 1 and Example 3 in the 1st fluid and the 2nd fluid, Fig. 4 is a graph showing the solubilities of Compound A in mediums with various pHs, and Fig. 5 is a graph showing the results of the dissolution test of the tablets prepared by the conventional formulation methods in the 1st fluid and the 2nd fluid.

### SUMMARY OF THE INVENTION

The object of this invention is to provide pharmaceutical compositions with improved dissolution of the Compound (I).

The present invention relates to pharmaceutical compositions prepared by mixing and pulverizing Compounds (I) with starch(es) derived from various plants and/or derivative(s) thereof at a weight ratio of from 1:4 to 2:1, preferably 1:2 to 2:1, followed by formulation thereof into preparations (hereinafter sometimes referred to as the Composition).

The present inventors have found that a sufficient dissolution-improving effect of Compounds (I) can be acknowledged and that the pharmaceutical preparations such as tablets, etc. comprising the Composition can be prepared into pharmaceutical preparations of practical sizes, which resulted in the completion of the invention.

### DETAILED DESCRIPTION OF THE INVENTION

In the above formula, R¹ and R² are the same or different and respectively mean hydrogen atom, halogen atom, trifluoromethyl, hydroxy, amino, nitro, cyano, C₁₋₄ alkyl, C₁₋₄ alkoxy or C₂₋₅ alkanoylamino, R³ means hydrogen atom, C₁₋₈ alkyl, hydroxy-C₁₋₄ alkyl, C₂₋₅ alkanoyloxy-C₁₋₄ alkyl, aryl, aryl-C₁₋₄ alkyl, heteroaryl, or aryl, aryl-C₁₋₄ alkyl or heteroaryl having 1 to 3 substituent(s) selected from halogen atom, trifluoromethyl, hydroxy, amino, nitro, cyano, C₁₋₄ alkyl, C₁₋₄ alkoxy and C₂₋₅ alkanoylamino on the aromatic ring, n means 0, 1 or 2, the bond ······· between the 4- and the 4a-positions means a single or double bond, when there is one asymmetric carbon atom in the formula, each optical isomer and racemic compound are encompassed, and when there are two or more asymmetric carbon atoms in the formula, each diastereomer (including each optical isomer and racemic compound) and mixtures of the diastereomers are encompassed.

Of the compounds of the formula (I), the preferred compounds are benzothiepino[5,4-c]pyridazine compounds wherein R¹ and R² are the same or different and are hydrogen atom, halogen atom or C₁₋₄ alkoxy, R³ is phenyl with or without halogen atom or C₁₋₄ alkoxy as a substituent, n means 0, 1 or 2, the bond ······· between the 4- and the 4a-positions means a single or double bond, when there is one asymmetric carbon atom in the formula, each optical isomer and racemic compound are encompassed, and when there are 2 or more asymmetric carbon atoms in the formula, each diastereomer (including each optical isomer and racemic compound) and mixtures of the diastereomers are encompassed.

Specifically preferred compounds are:
o 2-(4-chlorophenyl)-5,6-dihydro-(1)benzothiepino[5,4-c]pyridazine-3(2H)-one 7-oxide
o 2-(4-chlorophenyl)-5,6-dihydro-10-fluoro-(1)benzothiepino[5,4-c]pyridazine-3(2H)-one 7-oxide
o 9-chloro-2-(4-chlorophenyl)-5,6-dihydro-(1)benzothiepino[5,4-c]pyridazine-3(2H)-one 7-oxide
o 8-chloro-2-(4-chlorophenyl)-5,6-dihydro-(1)benzothiepino[5,4-c]pyridazine-3(2H)-one 7-oxide
o 10-chloro-2-(4-chlorophenyl)-5,6-dihydro-(1)benzothiepino[5,4-c]pyridazine-3(2H)-one 7-oxide
o 2-(4-bromophenyl)-5,6-dihydro-(1)benzothiepino[5,4-c]pyridazine-3(2H)-one 7-oxide
o 10-fluoro-2-(4-fluorophenyl)-5,6-dihydro-(1)benzothiepino[5,4-c]pyridazine-3(2H)-one 7-oxide
o 2-(4-chlorophenyl)-5,6-dihydro-(1)benzothiepino[5,4-c]pyridazine-3(2H)-one 7,7-dioxide
o 2-(4-chlorophenyl)-5,6-dihydro-(1)benzothiepino[5,4-c]pyridazine-3(2H)-one
o 2-(4-chlorophenyl)-4,4a,5,6-tetrahydro-(1)benzothiepino[5,4-c]pyridazine-3(2H)-one
o 2-(4-chlorophenyl)-5,6-dihydro-10-fluoro-(1)benzothiepino[5,4-c]pyridazine-3(2H)-one
o 2-(4-chlorophenyl)-5,6-dihydro-10-fluoro-(1)benzothiepino[5,4-c]pyridazine-3(2H)-one 7,7-dioxide
o 2-(4-chlorophenyl)-10-fluoro-4,4a,5,6-tetrahydro-(1)benzothiepino[5,4-c]pyridazine-3(2H)-one
o 2-(4-chlorophenyl)-10-fluoro-4,4a,5,6-tetrahydro-(1)benzothiepino[5,4-c]pyridazine-3(2H)-one 7-oxide
o 2-(4-chlorophenyl)-4,4a,5,6-tetrahydro-(1)benzothiepino[5,4-c]pyridazine-3(2H)-one 7-oxide
o 2-(4-methoxyphenyl)-5,6-dihydro-(1)benzothiepino[5,4-c]pyrid azine-3(2H)-one 7-oxide
o 2-(4-chlorophenyl)-10-methoxy-5,6-dihydro-(1)benzothiepino[5,4-c]pyridazine-3(2H)-one 7-oxide
o 2-(4-chlorophenyl)-9-methoxy-5,6-dihydro-(1)benzothiepino[5,4-c]pyridazine-3(2H)-one 7-oxide
o 8-chloro-2-(4-chlorophenyl)-5,6-dihydro-(1)benzothiepino[5,4-c]pyridazine-3(2H)-one
o 2-(4-methoxyphenyl)-5,6-dihydro-(1)benzothiepino[5,4-c]pyrid azine-3(2H)-one and
o 2-(4-methoxyphenyl)-5,6-dihydro-(1)benzothiepino[5,4-c]pyrid azine-3(2H)-one 7,7-dioxide
When these compounds contain one asymmetric carbon atom, each optical isomer and racemic compound are encompassed, and when there are 2 or more asymmetric carbon atoms in the formula, each diastereomer (including each optical isomer and racemic compound) and mixtures of the diastereomers are encompassed in the invention.

As the starches employed for the invention, there can be mentioned, for example, corn starch, potato starch, partially α-starch, etc., and as their derivatives, mention can be made of hydroxypropyl starch, sodium glycolate starch, phosphate starch, acetate starch, etc., all of which are known compounds as additives for pharmaceutical preparations.

In the present invention, one or more starches and their derivatives selected from those mentioned above may be used. Of the starches and their derivatives mentioned above, preferred are corn starch and potato starch.

According to the present invention, the mixing ratio of the Compound (I) and starch(es) derived from various plants and/or derivative(s) thereof is between 1:4 and 2:1 by weight, preferably between 1:2 and 2:1. If the Compound (I) is contained beyond the ratio of 2:1 (Compound content ca. 66%), the dissolution-improving effect of the Compound decreases. If the Compound (I) is contained below the ratio of 1:4, namely, starch(es) of various derivations is(are) contained beyond the ratio of 1:4 (starch content ca. 80%), the dosage form such as tablets, etc. becomes bulky and inadequate for administration.

For pulverizing the mixture, a pulverizing apparatus normally employed for formulation of pharmaceutical preparations such as atomizer, Jet Mill, hybridizer, Mechano-mill, Ball-mill, automatic mortar, etc. is used.

In the invention, the formulation includes addition of additives normally used for pharmaceutical preparations and preparation thereof into pharmaceutical preparations with volume and form convenient for administration.

Normally, the powders obtained by mixing Compound (I) and starch(es) derived from various plants and/or derivative(s) thereof at a weight ratio of between 1:4 and 2:1, preferably 1:2 and 2:1, and pulverizing the same (hereinafter referred merely as the mixed powders), are formulated into preparations after addition of additives normally used for pharmaceutical preparations. The additives normally used for pharmaceutical preparations mean binders, excipients, disintegrators, lubricants, or the like, on which no limitation is posed as long as they are pharmaceutically inactive and harmless. Examples of such additives include lactose, crystalline cellulose, water-soluble polymers such as polyvinylpyrrolidone, hydroxypropylcellulose, hydroxypropylmethylcellulose, methylcellulose, etc., low substituted hydroxypropylcellulose, Carmellose sodium, light anhydrous silicic acid, water-containing silicon dioxide, magnesium stearate, or the like. Normally, one or more additives mentioned above are added to the mixed powders. The additive(s) may be added prior to pulverization of the mixture of Compound (I) and starch(es) derived from various plants and/or derivative(s) thereof, which is encompassed in the present invention as long as the addition before the pulverization is not detrimental to the dissolution property of the Composition.

As the final formulation of the pharmaceutical compositions of the invention, there can be mentioned tablets (including sugar-coated tablets and film-coated tablets), powders (including granules, fine granules and/or those which are film-coated), capsules, or the like. A method known in the art may be adopted for the formulation of the preparations. The amount of the Compound contained in the final formulation using the Composition is 1-30 weight%, preferably 1-20 weight%.

The pharmaceutical composition of the present invention is vastly improved in the dissolution property in the 1st fluid (pH ca. 1.2) and the 2nd fluid (pH ca. 6.8) of the disintegration test method of the Japan Pharmacopoeia (hereinafter abbreviated as 1st fluid and 2nd fluid) as compared with preparations produced by a formulation method generally employed. It has been found that the pharmaceutical compositions dissolved in 1st fluid and 2nd fluid shows supersaturation of the Compound (I) and that the supersaturation is maintained for a long time. These findings imply prospect of improving the solubility of the Compound in the body after administration, and further, enhanced bioavailability.

The present invention is hereinbelow described by, but not limited to, reference examples and working examples. The compound employed was 2-(4-chlorophenyl)-5,6-dihydro-(1)benzothiepino[5,4-c]pyridazine-3(2H)-one 7-oxide (Compound A), but other compounds showed similar results as well.

| Reference Example 1 | |
|---|---|
| Compound A | 1.0% |
| potato starch | 2.0% |
| lactose | 96.5% |
| methylcellulose | 0.5% |

Compound A was pulverized by an atomizer to give fine powders having an average particle diameter of 10»m. The Compound A, lactose and potato starch were thoroughly mixed in a kneading machine, followed by addition of a methylcellulose paste and kneading. The kneaded preparation was passed through a sieve of 24 mesh to granulate, dried in an air forced oven at 50 °C till the water content became 3-4%, and thereafter passed through a sieve of 32 mesh to afford fine granules.

| Example 1 | |
|---|---|
| Compound A | 1.0% |
| potato starch | 2.0% |
| lactose | 96.5% |
| methylcellulose | 0.5% |

Compound A and potato starch were vigorously dry mixed in a mortar to give mixed and pulverized powders. The powders were mixed with lactose. The procedure of Reference Example 1 was followed to give fine granules.

The dissolution test was conducted with the fine granules of Reference Example 1 and Example 1 in accordance with the 2nd method of the dissolution test of the Japan Pharmacopoeia using 1st fluid and 2nd fluid of the disintegration test method of the Japan Pharmacopoeia (Paddle Method, 900 ml, 37°C, 100 rpm), the results of which are summarized in Fig. 1. The 1st fluid was prepared by adding dilute hydrochloric acid (24.0 ml) and water to sodium chloride (2.0 g) for dissolution to afford a 1000 ml solution (pH ca. 1.2). The 2nd fluid was prepared by adding 0.2N sodium hydroxide test solution (118 ml) and water to 0.2M potassium dihydrogenphosphate test solution (250 ml) to afford a 1000 ml solution (pH ca. 6.8). In the Figure, a and b respectively show the results of the dissolution test of the fine granules of Reference Example 1 and Example 1 in 1st fluid, and c and d respectively show the results of the dissolution test of the fine granules of Reference Example 1 and Example 1 in 2nd fluid.

The results show that the dissolution property was markedly improved by mixing and pulverizing Compound A and starch(es) of various derivations.

| Reference Example 2 | |
|---|---|
| Compound A | 10.0 mg |
| corn starch | 20.0 mg |
| lactose | 81.5 mg |
| crystalline cellulose | 30.0 mg |
| hydroxypropylcellulose | 3.0 mg |
| talc | 5.0 mg |
| magnesium stearate | 0.5 mg |

Compound A, corn starch, lactose and crystalline cellulose were mixed. Thereto was added a hydroxypropylcellulose paste and the mixture was kneaded for granulation. The granulated preparation was passed through a sieve of 16 mesh, dried in an air forced oven at 50 °C till the water content became 3-4% and passed through a sieve of 24 mesh. Talc and magnesium stearate were added thereto, and tablets of 150 mg per tablet were prepared by a rotary tablet machine.

| Example 2 | |
|---|---|
| Compound A | 10.0 mg |
| corn starch | 20.0 mg |
| lactose | 81.5 mg |
| crystalline cellulose | 30.0 mg |
| hydroxypropylcellulose | 3.0 mg |
| talc | 5.0 mg |
| magnesium stearate | 0.5 mg |

Compound A and potato starch were vigorously dry mixed in a mortar to give mixed and pulverized powders. The powders were mixed with lactose and crystalline cellulose. The procedure of Reference Example 2 was followed to give tablets.

The dissolution test as conducted with the fine granules of Reference Example 1 and Example 1 was conducted as regards the tablets of Reference Example 2 and Example 2, the results of which showing the superior dissolution-improving effect are summarized in Fig. 2. In the Figure, e and f respectively show the results of the dissolution test of the tablets of Reference Example 2 and Example 2 in 1st fluid, and g and h respectively show the results of the dissolution test of the tablets of Reference Example 2 and Example 2 in 2nd fluid.

| Example 3 | |
|---|---|
| Compound A | 1.0% |
| potato starch | 0.5% |
| lactose | 98.0% |
| methylcellulose | 0.5% |

Compound A and potato starch were treated by a hybridizer to give mixed and pulverized powders. The powders were mixed with lactose, and the same procedure as in Reference Example 1 was followed to give fine granules.

The dissolution test with the fine granules of Reference Example 1 and Example 3 was conducted in accordance with the 2nd method of the dissolution test of the Japan Pharmacopoeia using 1st fluid and 2nd fluid of the disintegration test method of the Japan Pharmacopoeia (Paddle Method, 900 ml, 37°C, 100 rpm), the results of which are summarized in Fig. 3. In the Figure, a and i respectively show the results of the dissolution test of the fine granules of Reference Example 1 and Example 3 in 1st fluid, and c and j respectively show the results of the dissolution test of the fine granules of Reference Example 1 and Example 3 in 2nd fluid.

The results show that the dissolution property is markedly improved by mixing and pulverizing Compound A and starch(es) of various derivations.

As regards the compounds other than Compound A, namely,
o 2-(4-chlorophenyl)-5,6-dihydro-10-fluoro-(1)benzothiepino[5,4-c]pyridazine-3(2H)-one 7-oxide
o 9-chloro-2-(4-chlorophenyl)-5,6-dihydro-(1)benzothiepino[5,4-c]pyridazine-3(2H)-one 7-oxide
o 8-chloro-2-(4-chlorophenyl)-5,6-dihydro-(1)benzothiepino[5,4-c]pyridazine-3(2H)-one 7-oxide
o 10-chloro-2-(4-chlorophenyl)-5,6-dihydro-(1)benzothiepino[5,4-c]pyridazine-3(2H)-one 7-oxide
o 2-(4-bromophenyl)-5,6-dihydro-(1)benzothiepino[5,4-c]pyridazine-3(2H)-one 7-oxide
o 10-fluoro-2-(4-fluorophenyl)-5,6-dihydro-(1)benzothiepino[5,4-c]pyridazine-3(2H)-one 7-oxide
o 2-(4-chlorophenyl)-5,6-dihydro-(1)benzothiepino[5,4-c]pyridazine-3(2H)-one 7,7-dioxide
o 2-(4-chlorophenyl)-5,6-dihydro-(1)benzothiepino[5,4-c]pyridazine-3(2H)-one
o 2-(4-chlorophenyl)-4,4a,5,6-tetrahydro-(1)benzothiepino[5,4-c]pyridazine-3(2H)-one
o 2-(4-chlorophenyl)-5,6-dihydro-10-fluoro-(1)benzothiepino[5,4-c]pyridazine-3(2H)-one
o 2-(4-chlorophenyl)-5,6-dihydro-10-fluoro-(1)benzothiepino[5,4-c]pyridazine-3(2H)-one 7,7-dioxide
o 2-(4-chlorophenyl)-10-fluoro-4,4a,5,6-tetrahydro-(1)benzothiepino[5,4-c]pyridazine-3(2H)-one
o 2-(4-chlorophenyl)-10-fluoro-4,4a,5,6-tetrahydro-(1)benzothiepino[5,4-c]pyridazine-3(2H)-one 7-oxide
o 2-(4-chlorophenyl)-4,4a,5,6-tetrahydro-(1)benzothiepino[5,4-c]pyridazine-3(2H)-one 7-oxide
o 2-(4-methoxyphenyl)-5,6-dihydro-(1)benzothiepino[5,4-c]pyrid azine-3(2H)-one 7-oxide
o 2-(4-chlorophenyl)-10-methoxy-5,6-dihydro-(1)benzothiepino[5,4-c]pyridazine-3(2H)-one 7-oxide
o 2-(4-chlorophenyl)-9-methoxy-5,6-dihydro-(1)benzothiepino[5,4-c]pyridazine-3(2H)-one 7-oxide
o 8-chloro-2-(4-chlorophenyl)-5,6-dihydro-(1)benzothiepino[5,4-c]pyridazine-3(2H)-one
o 2-(4-methoxyphenyl)-5,6-dihydro-(1)benzothiepino[5,4-c]pyrid azine-3(2H)-one and
o 2-(4-methoxyphenyl)-5,6-dihydro-(1)benzothiepino[5,4-c]pyrid azine-3(2H)-one 7,7-dioxide, the pharmaceutical
compositions were prepared according to Reference Examples and Examples described above, and subjected to the dissolution test.

The obtained results show similar dissolution-improving effect.

As discussed in the present specification, particularly in Examples, the pharmaceutical compositions with improved dissolution property can be obtained by mixing benzothiepino[5,4-c]pyridazine compounds (when there is one asymmetric carbon atom, each racemic compound and optical isomer are encompassed, and when there are two or more asymmetric carbon atoms, each diastereomer or mixtures thereof are encompassed) with starch(es) of various derivations and derivative(s) thereof, preferably one or more selected from corn starch, potato starch, partially α-starch, hydroxypropyl starch, sodium glycolate starch, phosphate starch, acetate starch, etc., more preferably corn starch and/or potato starch at a mixing ratio of from 1:4 to 2:1, preferably 1:2 to 2:1 by weight, and vigorously pulverizing the same with the use of a pulverizing apparatus such as a mortar, etc. followed by addition of additives normally employed for pharmaceutical preparations and formulation thereof into preparations.

The pharmaceutical compositions of the invention exhibit superior dissolution-improving effect accompanied by enhancement of the solubility of the Compound (I) per se as compared with that when the Compound alone is dissolved.

## Claims (Claims for the following Contracting State(s): AT, BE, CH, DE, FR, GB, IT, LI, NL, SE)

1. A pharmaceutical composition with improved dissolution property, which is obtained by mixing and pulverizing a benzothiepino[5,4-c]pyridazine compound represented by the formula 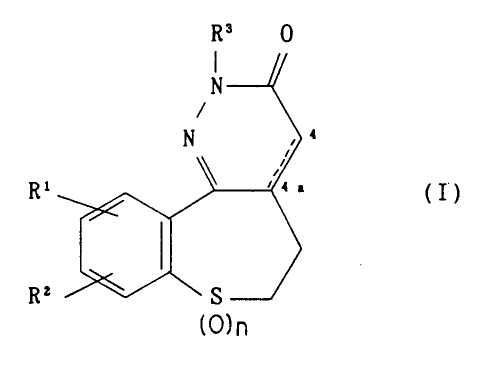 wherein R¹ and R² are the same or different and respectively mean hydrogen atom, halogen atom, trifluoromethyl, hydroxy, amino, nitro, cyano, C₁₋₄ alkyl, C₁₋₄ alkoxy or C₂₋₅ alkanoylamino, R³ means hydrogen atom, C₁₋₈ alkyl, hydroxy-C₁₋₄ alkyl, C₂₋₅ alkanoyloxy-C₁₋₄ alkyl, aryl, aryl-C₁₋₄ alkyl, heteroaryl, or aryl, aryl-C₁₋₄ alkyl or heteroaryl having 1 to 3 substituent(s) selected from the group consisting of halogen atom, trifluoromethyl, hydroxy, amino, nitro, cyano, C₁₋₄ alkyl, C₁₋₄ alkoxy and C₂₋₅ alkanoylamino on the aromatic ring, n means 0, 1 or 2, the bond ······· between the 4- and 4a-positions means a single or double bond, when there is one asymmetric carbon atom in the formula, each optical isomer and racemic compound are encompassed, and when there are two or more asymmetric carbon atoms in the formula, each diastereomer (including each optical isomer and racemic compound) and mixtures of the diastereomers are encompassed, with starch(es) derived from various plants and/or derivative(s) thereof at a weight ratio of from 1:4 to 2:1, followed by formulation thereof into preparations.

2. A pharmaceutical composition as claimed in claim 1, wherein the ratio of the benzothienino[5,4-c]pyridazine compound and the starch(es) and/or derivative(s) thereof is 1:2 to 2:1 by weight.

3. A pharmaceutical composition as claimed in claim 1, wherein the formulation comprises addition of additives normally used for pharmaceutical preparations.

4. A pharmaceutical composition as claimed in claim 1, in which the benzothiepino[5,4-c]pyridazine compound is that wherein R¹ and R² are the same or different and are hydrogen atom, halogen atom or C₁₋₄ alkoxy, R³ is phenyl with or without halogen atom or C₁₋₄ alkoxy as a substituent, n means 0, 1 or 2, the bond ······· between the 4- and the 4a-positions means a single or double bond, when there is one asymmetric carbon atom in the formula, each optical isomer and racemic compound are encompassed, and when there are two or more asymmetric carbon atoms in the formula, each diastereomer (including each optical isomer and racemic compound) and mixtures of the diastereomers are encompassed.

5. A pharmaceutical composition as claimed in claim 1, wherein the benzothiepino[5,4-c]pyridazine compound is selected from the group consisting of 2-(4-chlorophenyl)-5,6-dihydro-(1)benzothiepino[5,4-c]pyridazine-3(2H)-one 7-oxide, 2-(4-chlorophenyl)-5,6-dihydro-10-fluoro-(1)benzothiepino[5,4-c]pyridazine-3(2H)-one 7-oxide, 9-chloro-2-(4-chlorophenyl)-5,6-dihydro-(1)benzothiepino[5,4-c]pyridazine-3(2H)-one 7-oxide, 8-chloro-2-(4-chlorophenyl)-5,6-dihydro-(1)benzothiepino[5,4-c]pyridazine-3(2H)-one 7-oxide, 10-chloro-2-(4-chlorophenyl)-5,6-dihydro-(1)benzothiepino[5,4-c]pyridazine-3(2H)-one 7-oxide, 2-(4-bromophenyl)-5,6-dihydro-(1)benzothiepino[5,4-c]pyridazine-3(2H)-one 7-oxide, 10-fluoro-2-(4-fluorophenyl)-5,6-dihydro-(1)benzothiepino[5,4-c]pyridazine-3(2H)-one 7-oxide, 2-(4-chlorophenyl)-5,6-dihydro-(1)benzothiepino[5,4-c]pyridazine-3(2H)-one 7,7-dioxide, 2-(4-chlorophenyl)-5,6-dihydro-(1)benzothiepino[5,4-c]pyridazine-3(2H)-one, 2-(4-chlorophenyl)-4,4a,5,6-tetrahydro-(1)benzothiepino[5,4-c]pyridazine-3(2H)-one, 2-(4-chlorophenyl)-5,6-dihydro-10-fluoro-(1)benzothiepino[5,4-c]pyridazine-3(2H)-one, 2-(4-chlorophenyl)-5,6-dihydro-10-fluoro-(1)benzothiepino[5,4-c]pyridazine-3(2H)-one 7,7-dioxide, 2-(4-chlorophenyl)-10-fluoro-4,4a,5,6-tetrahydro-(1)benzothiepino[5,4-c]pyridazine-3(2H)-one, 2-(4-chlorophenyl)-10-fluoro-4,4a,5,6-tetrahydro-(1)benzothiepino[5,4-c]pyridazine-3(2H)-one 7-oxide, 2-(4-chlorophenyl)-4,4a,5,6-tetrahydro-(1)benzothiepino[5,4-c]pyridazine-3(2H)-one 7-oxide, 2-(4-methoxyphenyl)-5,6-dihydro-(1)benzothiepino[5,4-c]pyridazine-3(2H)-one 7-oxide, 2-(4-chlorophenyl)-10-methoxy-5,6-dihydro-(1)benzothiepino[5,4-c]pyridazine-3(2H)-one 7-oxide, 2-(4-chlorophenyl)-9-methoxy-5,6-dihydro-(1)benzothiepino[5,4-c]pyridazine-3(2H)-one 7-oxide, 8-chloro-2-(4-chlorophenyl)-5,6-dihydro-(1)benzothiepino[5,4-c]pyridazine-3(2H)-one, 2-(4-methoxyphenyl)-5,6-dihydro-(1)benzothiepino[5,4-c]pyridazine-3(2H)-one and 2-(4-methoxyphenyl)-5,6-dihydro-(1)benzothiepino[5,4-c]pyridazine-3(2H)-one 7,7-dioxide.

6. A pharmaceutical composition as claimed in claim 1, 2, 3, 4 or 5, wherein the starch(es) derived from various plants and/or derivative(s) thereof are one species or more selected from the group consisting of corn starch, potato starch, partially α-starch, sodium glycolate starch, phosphate starch and acetate starch.

7. A method for the preparation of a pharmaceutical composition, which comprises mixing and pulverizing a benzothiepino[5,4-c]pyridazine compound represented by the formula 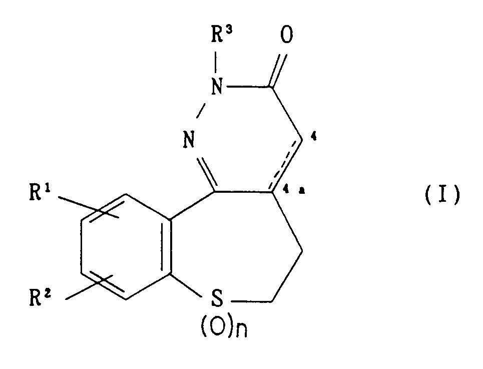 wherein R¹ and R² are the same or different and respectively mean hydrogen atom, halogen atom, trifluoromethyl, hydroxy, amino, nitro, cyano, C₁₋₄ alkyl, C₁₋₄ alkoxy or C₂₋₅ alkanoylamino, R³ means hydrogen atom, C₁₋₈ alkyl, hydroxy-C₁₋₄ alkyl, C₂₋₅ alkanoyloxy-C₁₋₄ alkyl, aryl, aryl-C₁₋₄ alkyl, heteroaryl, or aryl, aryl-C₁₋₄ alkyl or heteroaryl having 1 to 3 substituent(s) selected from the group consisting of halogen atom, trifluoromethyl, hydroxy, amino, nitro, cyano, C₁₋₄ alkyl, C₁₋₄ alkoxy and C₂₋₅ alkanoylamino on the aromatic ring, n means 0, 1 or 2, the bond ······· between the 4- and 4a-positions means a single or double bond, when there is one asymmetric carbon atom in the formula, each optical isomer and racemic compound are encompassed, and when there are two or more asymmetric carbon atoms in the formula, each diastereomer (including each optical isomer and racemic compound) and mixtures of the diastereomers are encompassed, with starch(es) derived from various plants and/or derivative(s) thereof at a weight ratio of from 1:4 to 2:1, followed by formulation of the powder obtained into pharmaceutical preparations.

## Claims (Claims for the following Contracting State(s): ES)

1. A method for the preparation of a pharmaceutical composition, which comprises mixing and pulverizing a benzothiepino[5,4-c]pyridazine compound represented by the formula 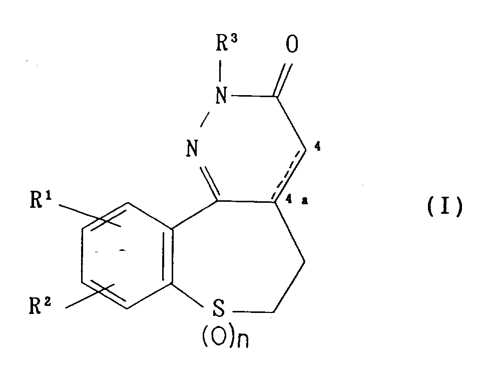 wherein R¹ and R² are the same or different and respectively mean hydrogen atom, halogen atom, trifluoromethyl, hydroxy, amino, nitro, cyano, C₁₋₄ alkyl, C₁₋₄ alkoxy or C₂₋₅ alkanoylamino, R³ means hydrogen atom, C₁₋₈ alkyl, hydroxy-C₁₋₄ alkyl, C₂₋₅ alkanoyloxy-C₁₋₄ alkyl, aryl, aryl-C₁₋₄ alkyl, heteroaryl, or aryl, aryl-C₁₋₄ alkyl or heteroaryl having 1 to 3 substituent(s) selected from the group consisting of halogen atom, trifluoromethyl, hydroxy, amino, nitro, cyano, C₁₋₄ alkyl, C₁₋₄ alkoxy and C₂₋₅ alkanoylamino on the aromatic ring, n means 0, 1 or 2, the bond ······· between the 4- and 4a-positions means a single or double bond, when there is one asymmetric carbon atom in the formula, each optical isomer and racemic compound are encompassed, and when there are two or more asymmetric carbon atoms in the formula, each diastereomer (including each optical isomer and racemic compound) and mixtures of the diastereomers are encompassed, with starch(es) derived from various plants and/or derivative(s) thereof at a weight ratio of from 1:4 to 2:1, followed by formulation of the powder obtained into pharmaceutical preparations.

2. A method as claimed in claim 1, wherein the ratio of the benzothienino[5,4-c]pyridazine compound and the starch(es) and/or derivative(s) thereof is 1:2 to 2:1 by weight.

3. A method as claimed in claim 1, wherein the formulation comprises addition of additives normally used for pharmaceutical preparations.

4. A method as claimed in claim 1, in which the benzothiepino[5,4-c]pyridazine compound is that wherein R¹ and R² are the same or different and are hydrogen atom, halogen atom or C₁₋₄ alkoxy, R³ is phenyl with or without halogen atom or C₁₋₄ alkoxy as a substituent, n means 0, 1 or 2, the bond ······· between the 4- and the 4a-positions means a single or double bond, when there is one asymmetric carbon atom in the formula, each optical isomer and racemic compound are encompassed, and when there are two or more asymmetric carbon atoms in the formula, each diastereomer (including each optical isomer and racemic compound) and mixtures of the diastereomers are encompassed.

5. A method as claimed in claim 1, wherein the benzothiepino[5,4-c]pyridazine compound is selected from the group consisting of 2-(4-chlorophenyl)-5,6-dihydro-(1)benzothiepino[5,4-c]pyridazine-3(2H)-one 7-oxide, 2-(4-chlorophenyl)-5_{,}6-dihydro-10-fluoro-(1)benzothiepino[5,4-c]pyridazine-3(2H)-one 7-oxide, 9-chloro-2-(4-chlorophenyl)-5,6-dihydro-(1)benzothiepino[5,4-c]pyridazine-3(2H)-one 7-oxide, 8-chloro-2-(4-chlorophenyl)-5,6-dihydro-(1)benzothiepino[5,4-c]pyridazine-3(2H)-one 7-oxide, 10-chloro-2-(4-chlorophenyl)-5,6-dihydro-(1)benzothiepino[5,4-c]pyridazine-3(2H)-one 7-oxide, 2-(4-bromophenyl)-5,6-dihydro-(1)benzothiepino[5,4-c]pyridazine-3(2H)-one 7-oxide, 10-fluoro-2-(4-fluorophenyl)-5,6-dihydro-(1)benzothiepino[5,4-c]pyridazine-3(2H)-one 7-oxide, 2-(4-chlorophenyl)-5,6-dihydro-(1)benzothiepino[5,4-c]pyridazine-3(2H)-one 7,7-dioxide, 2-(4-chlorophenyl)-5,6-dihydro-(1)benzothiepino[5,4-c]pyridazine-3(2H)-one 2-(4-chlorophenyl)-4,4a,5,6-tetrahydro-(1)benzothiepino[5,4-c]pyridazine-3(2H)-one, 2-(4-chlorophenyl)-5,6-dihydro-10-fluoro-(1)benzothiepino[5,4-c]pyridazine-3(2H)-one, 2-(4-chlorophenyl)-5,6-dihydro-10-fluoro-(1)benzothiepino[5,4-c]pyridazine-3(2H)-one 7,7-dioxide, 2-(4-chlorophenyl)-10-fluoro-4,4a,5,6-tetrahydro-(1)benzothiepino[5,4-c]pyridazine-3(2H)-one, 2-(4-chlorophenyl)-10-fluoro-4,4a,5,6-tetrahydro-(1)benzothiepino[5,4-c]pyridazine-3(2H)-one 7-oxide, 2-(4-chlorophenyl)-4,4a,5,6-tetrahydro-(1)benzothiepino[5,4-c]pyridazine-3(2H)-one 7-oxide, 2-(4-methoxyphenyl)-5,6-dihydro-(1)benzothiepino[5,4-c]pyridazine-3(2H)-one 7-oxide, 2-(4-chlorophenyl)-10-methoxy-5,6-dihydro-(1)benzothiepino[5,4-c]pyridazine-3(2H)-one 7-oxide, 2-(4-chlorophenyl)-9-methoxy-5,6-dihydro-(1)benzothiepino[5,4-c]pyridazine-3(2H)-one 7-oxide, 8-chloro-2-(4-chlorophenyl)-5,6-dihydro-(1)benzothiepino[5,4-c]pyridazine-3(2H)-one, 2-(4-methoxyphenyl)-5,6-dihydro-(1)benzothiepino[5,4-c]pyridazine-3(2H)-one and 2-(4-methoxyphenyl)-5,6-dihydro-(1)benzothiepino[5,4-c]pyridazine-3(2H)-one 7,7-dioxide.

6. A method as claimed in claim 1, 2, 3, 4 or 5, wherein the starch(es) derived from various plants and/or derivative(s) thereof are one species or more selected from the group consisting of corn starch, potato starch, partially α-starch, sodium glycolate starch, phosphate starch and acetate starch.

## Patentansprüche (Patentansprüche für folgende(n) Vertragsstaat(en): AT, BE, CH, DE, FR, GB, IT, LI, NL, SE)

1. Pharmazeutische Zusammensetzung mit verbesserten Auflösungseigenschaften, die erhalten wird durch Mischen und Pulverisieren einer Benzothiepino[5,4-c]pyridazinverbindung, die durch die Formel 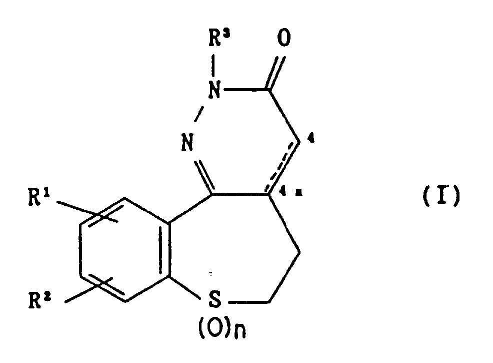 dargestellt wird, worin R¹ und R² gleich oder verschieden sind und jeweils ein Wasserstoffatom, Halogenatom, Tri-fluormethyl, Hydroxy, Amino, Nitro, Cyan, C₁₋₄-Alkyl C₁₋₄-Alkoxy oder C₂₋₅-Alkanoylamino bedeuten, R³ ein Wasserstoffatom, C₁₋₈-Alkyl, Hydroxy-C₁₋₄-alkyl, C₂₋₅-Alkanoyloxy-C₁₋₄-alkyl, Aryl, Aryl-C₁₋₄-alkyl, Heteroaryl oder Aryl, Aryl-C₁₋₄-alkyl oder Heteroaryl mit 1 bis 3 Substituenten, die aus der Gruppe ausgewählt sind, die aus Halogenatom, Tri-fluormethyl, Hydroxy, Amino, Nitro, Cyan C₁₋₄-Alkyl C₁₋₄-Alkoxy und C₂₋₅-Alkanoylamino besteht, am aromatischen Ring bedeutet, n 0, 1 oder 2 bedeutet, die Bindung ----- zwischen der 4- und 4a-Position eine Einfach- oder Doppelbindung bedeutet, wenn es in der Formel ein asymmetrisches Kohlenstoffatom gibt, jedes optische Isomer und die racemische Verbindung mit eingeschlossen sind, und wenn es in der Formel zwei oder mehr asymmetrische Kohlenstoffatome gibt, jedes Diastereomer (einschließlich jedem optischen Isomer und jeder racemischen Verbindung) und Gemische der Diastereomeren mit eingeschlossen sind,
mit Stärke(n), die von verschiedenen Pflanzen abgeleitet ist (sind), und/oder Derivaten davon in einem Gewichtsverhältnis von 1:4 bis 2:1 und anschließende Zubereitung des Gemischs zu Arzneipräparaten.

2. Pharmazeutische Zusammensetzung gemäß Anspruch 1, worin das Gewichtsverhältnis der Benzothiepino[5,4-c]pyridazinverbindung zu der (den) Stärke(n) und/oder den Derivaten davon 1:2 bis 2:1 beträgt.

3. Pharmazeutische Zusammensetzung gemäß Anspruch 1, worin die Zubereitung das Hinzufügen von Additiven, die normalerweise für pharmazeutische Präparate verwendet werden, umfaßt.

4. Pharmazeutische Zusammensetzung gemäß Anspruch 1, worin die Benzothiepino[5,4-c]pyridazinverbindung diejenige ist, in der R¹ und R² gleich oder verschieden sind und ein Wasserstoffatom, Halogenatom oder C₁₋₄-Alkoxy sind, R³ Phenyl mit oder ohne Halogenatom oder C₁₋₄-Alkoxy als Substituent ist, n 0, 1 oder 2 bedeutet, die Bindung ----- zwischen der 4- und 4a-Position eine Einfach- oder Doppelbindung bedeutet, wenn es in der Formel ein asymmetrisches Kohlenstoffatom gibt, jedes optische Isomer und die racemische Verbindung mit eingeschlossen sind, und wenn es in der Formel zwei oder mehr asymmetrische Kohlenstoffatome gibt, jedes Diastereomer (einschließlich jedem optischen Isomer und jeder racemischen Verbindung) und Gemische der Diastereomeren mit eingeschlossen sind.

5. Pharmazeutische Zusammensetzung gemäß Anspruch 1, worin die Benzothiepino[5,4-c]pyridazinverbindung aus der Gruppe ausgewählt wird, bestehend aus 2-(4-Chlorphenyl)-5,6-dihydro-(1)benzothiepino[5,4-c]pyridazin-3(2H)-on-7-oxid, 2-(4-Chlorphenyl)-5,6-dihydro-10-fluor-(1)benzothiepino[5,4-c] pyridazin-3(2H)-on-7-oxid, 9-Chlor-2-(4-chlorphenyl)-5,6-dihydro-(1)benzothiepino[5,4-c]pyridazin-3(2H)-on-7-oxid, 8-Chlor-2-(4-chlorphenyl)-5,6-dihydro-(1)benzothiepino[5,4-c]pyridazin-3(2H)-on-7-oxid, 10-Chlor-2-(4-chlorphenyl)-5,6-dihydro-(1)benzothiepino[5,4-c]pyridazin-3(2H)-on-7-oxid, 2-(4-Bromphenyl)-5,6-dihydro-(1)benzothiepino[5,4-c]pyridazin-3(2H)-on-7-oxid, 10-Fluor-2-(4-fluorphenyl)-5,6-dihydro-(1)benzothiepino[5,4-c]pyridazin-3(2H)-on-7-oxid, 2-(4-Chlorphenyl)-5,6-dihydro-(1)benzothiepino[5,4-c]pyridazin-3(2H)-on-7,7-dioxid, 2-(4-Chlorphenyl)-5,6-dihydro-(1)benzothiepino[5,4-c]pyridazin-3(2H)-on, 2-(4-Chlorphenyl)-4,4a,5,6-tetrahydro-(1)benzothiepino[5,4-c]pyridazin-3(2H)-on,2-(4-Chlorphenyl)-5,6-dihydro-10-fluor-(1)benzothiepino[5,4-c]pyridazin-3(2H)-on, 2-(4-Chlorphenyl)-5,6-dihydro-10-fluor-(1)benzothiepino[5,4-c]pyridazin-3(2H)-on-7,7-dioxid, 2-(4-Chlorphenyl)-10-fluor-4,4a,5,6-tetrahydro-(1)benzothiepino[5,4-c]pyridazin-3(2H)-on,2-(4-Chlorphenyl)-10-fluor-4,4a,5,6-tetrahydro-(1)benzothiepino[5,4-c]pyridazin-3(2H)-on-7-oxid, 2-(4-Chlorphenyl)-4,4a,5,6-tetrahydro-(1)benzothiepino[5,4-c]pyridazin-3(2H)-on-7-oxid, 2-(4-Methoxyphenyl)-5,6-dihydro-(1)benzothiepino[5,4-c]pyridazin-3(2H)-on-7-oxid, 2-(4-Chlorphenyl)-10-methoxy-5,6-dihydro-(1)benzothiepino[5,4-c]pyridazin-3(2H)-on-7-oxid, 2-(4-Chlorphenyl)-9-methoxy-5,6-dihydro-(1)benzothiepino[5,4-c]pyridazin-3(2H)-on-7-oxid, 8-Chlor-2-(4-chlorphenyl)-5,6-dihydro-(1)benzothiepino[5,4-c]pyridazin-3(2H)-on,2-(4-Methoxyphenyl)-5,6-dihydro-(1)benzothiepino[5,4-c]pyridazin-3(2H)-on und 2-(4-Methoxyphenyl)-5,6-dihydro-(1)benzothiepino[5,4-c]pyridazin-3(2H)-on-7,7-dioxid.

6. Pharmazeutische Zusammensetzung gemäß Anspruch 1, 2, 3, 4 oder 5, worin die von verschiedenen Pflanzen abgeleitete(n) Stärke(n) und/oder Derivate davon eine oder mehrere Spezies sind, die aus der Gruppe ausgewählt sind, die aus Maisstärke, Kartoffelstärke, Teil-α-Stärke, Natriumglycolatstärke, Phosphatstärke und Acetatstärke besteht.

7. Verfahren zur Herstellung einer pharmazeutischen Zusammensetzung, umfassend das Mischen und Pulverisieren einer Benzothiepino[5,4-c]pyridazinverbindung, die durch die Formel 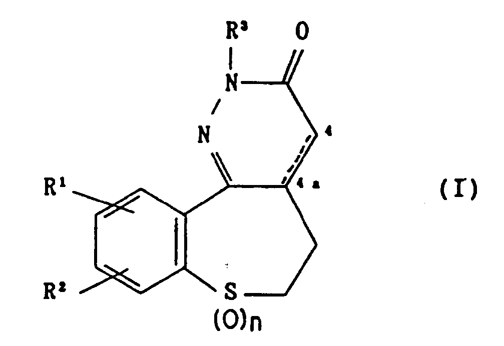 dargestellt wird, worin R¹ und R² gleich oder verschieden sind und jeweils ein Wasserstoffatom, Halogenatom, Tri-fluormethyl, Hydroxy, Amino, Nitro, Cyan, C₁₋₄-Alkyl, C₁₋₄-Alkoxy oder C₂₋₅-Alkanoylamino bedeuten, R³ ein Wasserstoffatom C₁₋₈-Alkyl, Hydroxy-C₁₋₄-alkyl, C₂₋₅-Alkanoyloxy-C₁₋₄-alkyl, Aryl, Aryl-C₁₋₄-alkyl, Heteroaryl oder Aryl, Aryl-C₁₋₄-alkyl oder Heteroaryl mit 1 bis 3 Substituenten, die aus der Gruppe ausgewählt sind, die aus Halogenatom, Trifluormethyl, Hydroxy, Amino, Nitro, Cyan, C₁₋₄-Alkyl, C₁₋₄-Alkoxy und C₂₋₅-Alkanoylamino besteht, am aromatischen Ring bedeutet, n 0, 1 oder 2 bedeutet, die Bindung ----- zwischen der 4- und 4a-Position eine Einfach- oder Doppelbindung bedeutet, wenn es in der Formel ein asymmetrisches Kohlenstoffatom gibt, jedes optische Isomer und die racemische Verbindung mit eingeschlossen sind, und wenn es in der Formel zwei oder mehr asymmetrische Kohlenstoffatome gibt, jedes Diastereomer (einschließlich jedem optischen Isomer und jeder racemischen Verbindung) und Gemische der Diastereomeren mit eingeschlossen sind,
mit Stärke(n), die von verschiedenen Pflanzen abgeleitet ist (sind), und/oder Derivaten davon in einem Gewichtsverhältnis von 1:4 bis 2:1 und anschließende Zubereitung des erhaltenen Pulvers zu pharmazeutischen Präparaten.

## Patentansprüche (Patentansprüche für folgende(n) Vertragsstaat(en): ES)

1. Verfahren zur Herstellung einer pharmazeutischen Zusammensetzung, umfassend das Mischen und Pulverisieren einer Benzothiepino[5,4-c]pyridazinverbindung, die durch die Formel 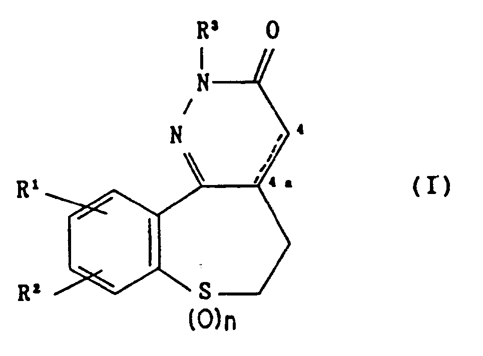 dargestellt wird, worin R¹ und R² gleich oder verschieden sind und jeweils ein Wasserstoffatom, Halogenatom, Tri-fluormethyl, Hydroxy, Amino, Nitro, Cyan, C₁₋₄-Alkyl, C₁₋₄-Alkoxy oder C₂₋₅-Alkanoylamino bedeuten, R³ ein Wasserstoffatom C₁₋₈-Alkyl, Hydroxy-C₁₋₄-alkyl, C₂₋₅-Alkanoyloxy-C₁₋₄-alkyl, Aryl, Aryl-C₁₋₄-alkyl, Heteroaryl oder Aryl, Aryl-C₁₋₄-alkyl oder Heteroaryl mit 1 bis 3 Substituenten, die aus der Gruppe ausgewählt sind, die aus Halogenatom, Tri-fluormethyl, Hydroxy, Amino, Nitro, Cyan, C₁₋₄-Alkyl C₁₋₄-Alkoxy und C₂₋₅-Alkanoylamino besteht, am aromatischen Ring bedeutet, n 0, 1 oder 2 bedeutet, die Bindung ----- zwischen der 4- und 4a-Position eine Einfach- oder Doppelbindung bedeutet, wenn es in der Formel ein asymmetrisches Kohlenstoffatom gibt, jedes optische Isomer und die racemische Verbindung mit eingeschlossen sind, und wenn es in der Formel zwei oder mehr asymmetrische Kohlenstoffatome gibt, jedes Diastereomer (einschließlich jedem optischen Isomer und jeder racemischen Verbindung) und Gemische der Diastereomeren mit eingeschlossen sind,
mit Stärke(n), die von verschiedenen Pflanzen abgeleitet ist (sind), und/oder Derivaten davon in einem Gewichtsverhältnis von 1:4 bis 2:1 und anschließende Zubereitung des erhaltenen Pulvers zu pharmazeutischen Präparaten.

2. Verfahren gemäß Anspruch 1, wobei das Gewichtsverhältnis der Benzothiepino[5,4-c]pyridazinverbindung zu der (den) Stärke(n) und/oder den Derivaten davon 1:2 bis 2:1 beträgt.

3. Verfahren gemäß Anspruch 1, wobei die Zubereitung das Hinzufügen von Additiven, die normalerweise für pharmazeutische Präparate verwendet werden, umfaßt.

4. Verfahren gemäß Anspruch 1, wobei die Benzothiepino [5,4-c]pyridazinverbindung diejenige ist, in der R¹ und R² gleich oder verschieden sind und ein Wasserstoffatom, Halogenatom oder C₁₋₄-Alkoxy sind, R³ Phenyl mit oder ohne Halogenatom oder C₁₋₄-Alkoxy als Substituent ist, n 0, 1 oder 2 bedeutet, die Bindung ----- zwischen der 4- und 4a-Position eine Einfach- oder Doppelbindung bedeutet, wenn es in der Formel ein asymmetrisches Kohlenstoffatom gibt, jedes optische Isomer und die racemische Verbindung mit eingeschlossen sind, und wenn es in der Formel zwei oder mehr asymmetrische Kohlenstoffatome gibt, jedes Diastereomer (einschließlich jedem optischen Isomer und jeder racemischen Verbindung) und Gemische der Diastereomeren mit eingeschlossen sind.

5. Verfahren gemäß Anspruch 1, wobei die Benzothiepino[5,4-c]pyridazinverbindung aus der Gruppe ausgewählt wird, bestehend aus 2-(4-Chlorphenyl)-5,6-dihydro-(1)benzothiepino[5,4-c]pyridazin-3(2H)-on-7-oxid,2-(4-Chlorphenyl)-5,6-di hydro-10-fluor-(1)benzothiepino[5,4-c]pyridazin-3(2H)-on-7-oxid,9-Chlor-2-(4-chlorphenyl)-5,6-dihydro-(1)benzothiepino[5,4-c]pyridazin-3(2H)-on-7-oxid,8-Chlor-2-(4-chlorphenyl)-5,6-dihydro-(1)benzothiepino[5,4-c]pyridazin-3(2H)-on-7-oxid, 10-Chlor-2-(4-chlorphenyl)-5,6-dihydro-(1)benzothiepino[5,4-c]pyridazin-3(2H)-on-7-oxid,2-(4-Bromphenyl)-5,6-dihydro-(1)benzothiepino[5,4-c]pyridazin-3(2H)-on-7-oxid, 10-Fluor-2-(4-fluorphenyl)-5,6-dihydro-(1)benzothiepino[5,4-c]pyridazin-3(2H)-on-7-oxid, 2-(4-Chlorphenyl)-5,6-dihydro-(1)benzothiepino[5,4-c]pyridazin-3(2H)-on-7,7-dioxid,2-(4-Chlorphenyl)-5,6-dihydro-(1)benzothiepino[5,4-c]pyridazin-3(2H)-on, 2-(4-Chlorphenyl)-4,4a,5,6-tetrahydro-(1)benzothiepino[5,4-c]pyridazin-3(2H)-on, 2-(4-Chlorphenyl)-5,6-dihydro-10-fluor-(1)benzothiepino[5,4-c]pyridazin-3(2H)-on, 2-(4-Chlorphenyl)-5,6-dihydro-10-fluor-(1)benzothiepino[5,4-c]pyridazin-3(2H)-on-7,7-dioxid,2-(4-Chlorphenyl)-10-fluor-4,4a,5,6-tetrahydro-(1)benzothiepino[5,4-c]pyridazin-3(2H)-on, 2-(4-Chlorphenyl)-10-fluor-4,4a,5,6-tetrahydro-(1)benzothiepino[5,4-c]pyridazin-3(2H)-on-7-oxid, 2-(4-Chlorphenyl)-4,4a,5,6-tetrahydro-(1)benzothiepino[5,4-c]pyridazin-3(2H)-on-7-oxid, 2-(4-Methoxyphenyl)-5,6-dihydro-(1)benzothiepino[5,4-c]pyridazin-3(2H)-on-7-oxid,2-(4-Chlorphenyl)-10-methoxy-5,6-dihydro-(1)benzothiepino[5,4-c]pyridazin-3(2H)-on-7-oxid,2-(4-Chlorphenyl)-9-methoxy-5,6-dihydro-(1)benzothiepino[5,4-c]pyridazin-3(2H)-on-7-oxid, 8-Chlor-2-(4-chlorphenyl)-5,6-dihydro-(1)benzothiepino[5,4-c]pyridazin-3(2H)-on, 2-(4-Methoxyphenyl)-5,6-dihydro-(1)benzothiepino[5,4-c]pyridazin-3(2H)-on und 2-(4-Methoxyphenyl)-5,6-dihydro-(1)benzothiepino[5,4-c]pyridazin-3(2H)-on-7,7-dioxid.

6. Verfahren gemäß Anspruch 1, 2, 3, 4 oder 5, worin die von verschiedenen pflanzen abgeleitete(n) Stärke(n) und/oder Derivate davon eine oder mehrere Spezies sind, die aus der Gruppe ausgewählt sind, die aus Maisstärke, Kartoffelstärke, Teil-α-Stärke, Natriumglycolatstärke, Phosphatstärke und Acetatstärke besteht.

## Revendications (Revendications pour l'(les) Etat(s) contractant(s) suivant(s): AT, BE, CH, DE, FR, GB, IT, LI, NL, SE)

1. Composition pharmaceutique ayant une propriété de dissolution améliorée, que l'on obtient par mélange et pulvérisation d'un composé benzothiépino[5,4-c]pyridazine représenté par la formule 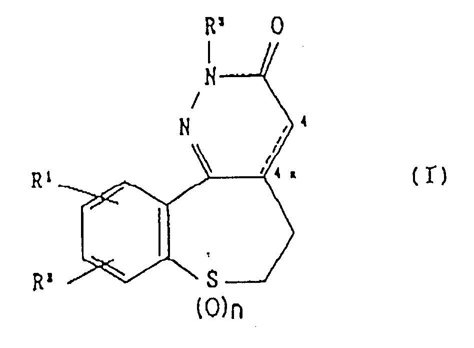 dans laquelle R¹ et R² sont identiques ou différents et représentent respectivement un atome d'hydrogène, un atome d'halogène, ou un groupe trifluorométhyle, hydroxy, amino, nitro, cyano, alkyle en C₁-C₄, alcoxy en C₁-C₄ ou alcanoylamino en C₂-C₅, R³ représente un atome d'hydrogène, ou un groupe alkyle en C₁-C₈, hydroxyalkyle en C₁-C₄, alcanoyloxy(en C₂-C₅)alkyle (en C₁-C₄), aryle, aryl(alkyle en C₁-C₄), hétéroaryle ou aryle, aryl(alkyle en C₁-C₄) ou hétéroaryle comportant 1 à 3 substitants choisis dans le groupe constitué par un atome d'halogène, ou un groupe trifluorométhyle, hydroxy, amino, nitro, cyano, alkyle en C₁-C₄, alcoxy en C₁-C₄ et alcanoylamino en C₂-C₅ sur le noyau aromatique, n représente 0, 1 ou 2, la liaison ----- entre les positions 4 et 4a représente une simple ou une double liaison, lorsqu'il existe dans la formule un atome de carbone asymétrique, chaque isomère optique et composé racémique est englobé et, lorsqu'il existe dans la formule au moins deux atomes de carbone asymétriques, chaque diastéréoisomère (y compris chaque composé isomère optique et racémique) et les mélanges des diastéréoisomères sont englobés, avec un ou des amidons dérivés de différentes plantes et/ou un ou des dérivés de celles-ci, dans un rapport pondéral de 1:4 à 2:1, suivie de sa formulation en préparations.

2. Composition pharmaceutique selon la revendication 1, dans laquelle le rapport du composé benzothiépino[5,4-c]pyridazine et du ou des amidons et/ou du ou des dérivés de ceux-ci est de 1:2 à 2:1 en poids.

3. Composition pharmaceutique selon la revendication 1, dans laquelle la formulation comprend l'addition d'additifs normalement utilisés pour les préparations pharmaceutiques.

4. Composition pharmaceutique selon la revendication 1, dans laquelle le composé benzothiépino[5,4-c]pyridazine est celui dans lequel R¹ et R² sont identiques ou différents et sont des atomes d'hydrogène, des atomes d'halogène ou des groupes alcoxy en C₁-C₄, R³ est un groupe phényle, avec ou sans atome d'halogène ou groupe alcoxy en C₁-C₄ comme substituant, n représente 0, 1 ou 2, la liaison ----- entre les positions 4 et 4a représente une simple ou une double liaison, lorsqu'il existe dans la formule un atome de carbone asymétrique, chaque isomère optique et composé racémique est englobé et, lorsqu'il existe dans la formule au moins deux atomes de carbone asymétriques, chaque diastéréoisomère (y compris chaque composé isomère optique et racémique) et les mélanges des diastéréoisomères sont englobés.

5. Composition pharmaceutique selon la revendication 1, dans laquelle le composé benzothiépino[5,4-c]pyridazine est choisi dans le groupe constitué par le 7-oxyde de 2-(4-chlorophényl)-5,6-dihydro-(1)benzothiépino[5,4-c]pyridazine-3(2H)-one, le 7-oxyde de 2-(4-chlorophényl)-5,6-dihydro-10-fluoro-(1)benzothiépino[5,4-c]pyridazine-3(2H)-one, le 7-oxyde de 9-chloro-2-(4-chlorophényl)-5,6-dihydro-(1)benzothiépino[5,4-c]pyridazine-3(2H)-one, le 7-oxyde de 8-chloro-2-(4-chlorophényl)-5,6-dihydro-(1)benzothiépino[5,4-c]pyridazine-3(2H)-one, le 7-oxyde de 10-chloro-2-(4-chlorophényl)-5,6-dihydro-(1)benzothiépino[5,4-c]pyridazine-3(2H)- one, le 7-oxyde de 2-(4-bromophényl)-5,6-dihydro-(1)benzothiépino[5,4-c]pyridazine-3(2H)-one, le 7-oxyde de 10-fluoro-2-(4-fluorophényl)-5,6-dihydro-(1)benzothiépino[5,4-c]pyridazine-3(2H)-one, le 7,7-dioxyde de 2-(4-chlorophényl)-5,6-dihydro-(1)benzothiépino[5,4-c]pyridazine-3(2H)-one, la 2-(4-chlorophényl)-5,6-dihydro-(1)benzothiépino[5,4-c]pyridazine-3(2H)-one, la 2-(4-chlorophényl)-4,4a,5,6-tétrahydro-(1)benzothiépino[5,4-c]pyridazine-3(2H)-one, la 2-(4-chlorophényl)-5,6-dihydro-10-fluoro-(1)benzothiépino[5,4-c]pyridazine-3(2H)-one, le 7,7-dioxyde de 2-(4-chlorophényl)-5,6-dihydro-10-fluoro-(1)benzothiépino[5,4-c]pyridazine-3(2H)-one, la 2-(4-chlorophényl)-10-fluoro-4,4a,5,6-tétrahydro-(1)benzothiépino[5,4-c]pyridazine-3(2H)-one, le 7-oxyde de 2-(4-chlorophényl)-10-fluoro-4,4a,5,6-tétrahydro-(1)benzothiépino[5,4-c]pyridazine-3(2H)-one, le 7-oxyde de 2-(4-chlorophényl)-4,4a,5,6-tétrahydro-(1)benzothiépino[5,4-c]pyridazine-3(2H)-one, le 7-oxyde de 2-(4-méthoxyphényl)-5,6-dihydro-(1)benzothiépino[5,4-c]pyridazine-3(2H)-one, le 7-oxyde de 2-(4-chlorophényl)-10-méthoxy-5,6-dihydro-(1)benzothiépino[5,4-c]pyridazine-3(2H)-one, le 7-oxyde de 2-(4-chlorophényl)-9-méthoxy-5,6-dihydro-(1)benzothiépino[5,4-c]pyridazine-3(2H)-one, la 8-chloro-2-(4-chlorophényl)-5,6-dihydro-(1)benzothiépino[5,4-c]pyridazine-3(2H)-one, la 2-(4-méthoxyphényl)-5,6-dihydro-(1)benzothiépino[5,4-c]pyridazine-3(2H)-one et le 7,7-dioxyde de 2-(4-méthoxyphényl)-5,6-dihydro-(1)benzothiépino[5,4-c]pyridazine-3(2H)-one.

6. Composition pharmaceutique selon la revendication 1, 2, 3, 4 ou 5, dans laquelle le ou les amidons dérivés de différentes plantes et/ou de leurs dérives sont une ou plusieurs espèces choisies dans le groupe constitué par l'amidon de maïs, la fécule de pomme de terre, l'amidon partiellement α, le glycolate de sodium-amidon, le phosphate-amidon et l'acétate-amidon.

7. Procédé de préparation d'une composition pharmaceutique, qui comprend le mélange et la pulvérisation d'un composé benzothiépino[5,4-c]pyridazine représenté par la formule 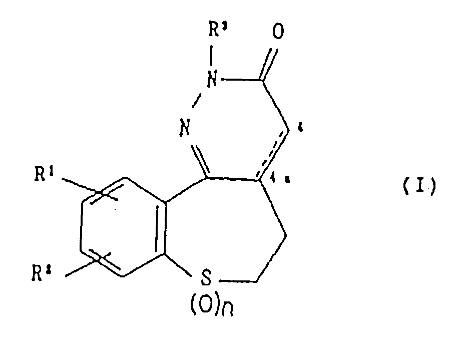 dans laquelle R¹ et R² sont identiques ou différents et représentent respectivement un atome d'hydrogène, un atome d'halogène, ou un groupe trifluorométhyle, hydroxy, amino, nitro, cyano, alkyle en C₁-C₄, alcoxy en C₁-C₄ ou alcanoylamino en C₂-C₅, R³ représente un atome d'hydrogène, ou un groupe alkyle en C₁-C₈, hydroxyalkyle en C₁-C₄, alcanoyloxy(en C₂-C₅)alkyle (en C₁-C₄), aryle, aryl(alkyle en C₁-C₄), hétéroaryle ou aryle, aryl(alkyle en C₁-C₄) ou hétéroaryle comportant 1 à 3 substitants choisis dans le groupe constitué par un atome d'halogène, ou un groupe trifluorométhyle, hydroxy, amino, nitro, cyano, alkyle en C₁-C₄, alcoxy en C₁-C₄ et alcanoylamino en C₂-C₅ sur le noyau aromatique, n représente 0, 1 ou 2, la liaison ----- entre les positions 4 et 4a représente une simple ou une double liaison, lorsqu'il existe dans la formule un atome de carbone asymétrique, chaque isomère optique et composé racémique est englobé et, lorsqu'il existe dans la formule au moins deux atomes de carbone asymétriques, chaque diastéréoisomère (y compris chaque composé isomère optique et racémique) et les mélanges des diastéréoisomères sont englobés, avec un ou des amidons dérivés de différentes plantes et/ou un ou des dérivés de celles-ci, dans un rapport pondéral de 1:4 à 2:1, suivie de la formulation de préparations pharmaceutiques à partir de la poudre obtenue.

## Revendications (Revendications pour l'(les) Etat(s) contractant(s) suivant(s): ES)

1. Procédé de préparation d'une composition pharmaceutique, qui comprend le mélange et la pulvérisation d'un composé benzothiépino[5,4-c]pyridazine représenté par la formule 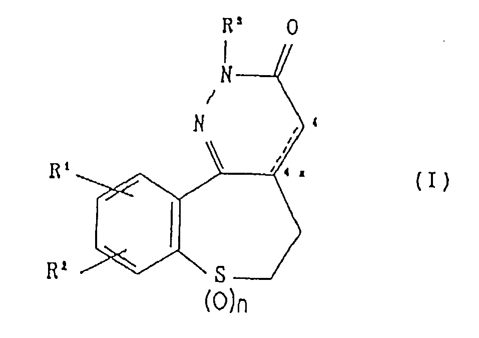 dans laquelle R¹ et R² sont identiques ou différents et représentent respectivement un atome d'hydrogène, un atome d'halogène, ou un groupe trifluorométhyle, hydroxy, amino, nitro, cyano, alkyle en C₁-C₄, alcoxy en C₁-C₄ ou alcanoylamino en C₂-C₅, R³ représente un atome d'hydrogène, ou un groupe alkyle en C₁-C₈, hydroxyalkyle en C₁-C₄, alcanoyloxy(en C₂-C₅)alkyle (en C₁-C₄), aryle, aryl(alkyle en C₁-C₄), hétéroaryle ou aryle, aryl(alkyle en C₁-C₄) ou hétéroaryle comportant 1 à 3 substitants choisis dans le groupe constitué par un atome d'halogène, ou un groupe trifluorométhyle, hydroxy, amino, nitro, cyano, alkyle en C₁-C₄, alcoxy en C₁-C₄ et alcanoylamino en C₂-C₅ sur le noyau aromatique, n représente 0, 1 ou 2, la liaison ----- entre les positions 4 et 4a représente une simple ou une double liaison, lorsqu'il existe dans la formule un atome de carbone asymétrique, chaque isomère optique et composé racémique est englobé et, lorsqu'il existe dans la formule au moins deux atomes de carbone asymétriques, chaque diastéréoisomère (y compris chaque composé isomère optique et racémique) et les mélanges des diastéréoisomères sont englobés, avec un ou des amidons dérivés de différentes plantes et/ou un ou des dérivés de celles-ci, dans un rapport pondéral de 1:4 à 2:1, suivie de la formulation de préparations pharmaceutiques à partir de la poudre obtenue.

2. Procédé selon la revendication 1, dans lequel le rapport du composé benzothiépino[5,4-c]pyridazine et du ou des amidons et/ou du ou des dérivés de ceux-ci est de 1:2 à 2:1 en poids.

3. Procédé selon la revendication 1, dans lequel la formulation comprend l'addition d'additifs normalement utilisés pour les préparations pharmaceutiques.

4. Procédé selon la revendication 1, dans lequel le composé benzothiépino[5,4-c]pyridazine est celui dans lequel R¹ et R² sont identiques ou différents et sont des atomes d'hydrogène, des atomes d'halogène ou des groupes alcoxy en C₁-C₄, R³ est un groupe phényle, avec ou sans atome d'halogène ou groupe alcoxy en C₁-C₄ comme substituant, n représente 0, 1 ou 2, la liaison ----- entre les positions 4 et 4a représente une simple ou une double liaison, lorsqu'il existe dans la formule un atome de carbone asymétrique, chaque isomère optique et composé racémique est englobé et, lorsqu'il existe dans la formule au moins deux atomes de carbone asymétriques, chaque diastéréoisomère (y compris chaque composé isomère optique et racémique) et les mélanges des diastéréoisomères sont englobés.

5. Procédé selon la revendication 1, dans lequel le composé benzothiépino[5,4-c]pyridazine est choisi dans le groupe constitué par le 7-oxyde de 2-(4-chlorophényl)-5,6-dihydro-(1)benzothiépino[5,4-c]pyridazine-3(2H)-one, le 7-oxyde de 2-(4-chlorophényl)-5,6-dihydro-10-fluoro-(1)benzothiépino[5,4-c]pyridazine-3(2H)-one, le 7-oxyde de 9-chloro-2-(4-chlorophényl)-5,6-dihydro-(1)benzothiépino[5,4-c]pyridazine-3(2H)-one, le 7-oxyde de 8-chloro-2-(4-chlorophényl)-5,6-dihydro-(1)benzothiépino[5,4-c]pyridazine-3(2H)-one, le 7-oxyde de 10-chloro-2-(4-chlorophényl)-5,6-dihydro-(1)benzothiépino[5,4-c]pyridazine-3(2H)-one, le 7-oxyde de 2-(4-bromophényl)-5,6-dihydro-(1)benzothiépino[5,4-c]pyridazine-3(2H)-one, le 7-oxyde de 10-fluoro-2-(4-fluorophényl)-5,6-dihydro-(1)benzothiépino[5,4-c]pyridazine-3(2H)-one, le 7,7-dioxyde de 2-(4-chlorophényl)-5,6-dihydro-(1)benzothiépino[5,4-c]pyridazine-3(2H)-one, la 2-(4-chlorophényl)-5,6-dihydro-(1)benzothiépino[5,4-c]pyridazine-3(2H)-one, la 2-(4-chlorophényl)-4,4a,5,6-tétrahydro-(1)benzothiépino[5,4-c]pyridazine-3(2H)-one, la 2-(4-chlorophényl)-5,6-dihydro-10-fluoro-(1)benzothiépino[5,4-c]pyridazine-3(2H)-one, le 7,7-dioxyde de 2-(4-chlorophényl)-5,6-dihydro-10-fluoro-(1)benzothiépino[5,4-c]pyridazine-3(2H)-one, la 2-(4-chlorophényl)-10-fluoro-4,4a,5,6-tétrahydro-(1)benzothiépino[5,4-c]pyridazine-3(2H)-one, le 7-oxyde de 2-(4-chlorophényl)-10-fluoro-4,4a,5,6-tétrahydro-(1)benzothiépino[5,4-c]pyridazine-3(2H)-one, le 7-oxyde de 2-(4-chlorophényl)-4,4a,5,6-tétrahydro-(1)benzothiépino[5,4-c]pyridazine-3(2H)-one, le 7-oxyde de 2-(4-méthoxyphényl)-5,6-dihydro-(1)benzothiépino[5,4-c]pyridazine-3(2H)-one, le 7-oxyde de 2-(4-chlorophényl)-10-méthoxy-5,6-dihydro-(1)benzothiépino[5,4-c]pyridazine-3(2H)-one, le 7-oxyde de 2-(4-chlorophényl)-9-méthoxy-5,6-dihydro-(1)benzothiépino[5,4-c]pyridazine-3(2H)-one, la 8-chloro-2-(4-chlorophényl)-5,6-dihydro-(1)benzothiépino[5,4-c]pyridazine-3(2H)-one, la 2-(4-méthoxyphényl)-5,6-dihydro-(1)benzothiépino[5,4-c]pyridazine-3(2H)-one et le 7,7-dioxyde de 2-(4-méthoxyphényl)-5,6-dihydro-(1)benzothiépino[5,4-c]pyridazine-3(2H)-one.

6. Procédé selon la revendication 1, 2, 3, 4 ou 5, dans lequel le ou les amidons dérivés de différentes plantes et/ou de leurs dérivés sont une ou plusieurs espèces choisies dans le groupe constitué par l'amidon de maïs, la fécule de pomme de terre, l'amidon partiellement α, le glycolate de sodium-amidon, le phosphate-amidon et l'acétate-amidon.
